# EUROPEAN PATENT SPECIFICATION

(11) **EP 1 089 718 B1**
(45) Date of publication and mention of the grant of the patent: **28.01.2004**
(21) Application number: 98934990.7
(22) Date of filing: 22.06.1998
(51) Int. Cl.: A61K 9/70, A61K 38/21

(54) **STICKING PLASTER FOR CONTROLLED RELEASE OF NATURAL INTERFERON**
SELBSTKLEBENDES PFLASTER ZUR GESTEUERTEN FREISETZUNG NATÜRLICHEN INTERFERONS
EMPLATRE ADHESIF POUR LA LIBERATION CONTROLEE DE L'INTERFERON NATUREL

(43) Date of publication of application: 11.04.2001
(73) Proprietor: Unihart Corporation, Dublin 2 (IE)
(72) Inventor: TARRO, Giulio, Ist. Farmacoterapico Italiano S.p.a, 00197 Rome (IT); BROZZO, Renzo, Ist. Farmacoterapico Italiano S.p.a, 00197 Rome (IT)
(74) Representative: Bianchetti, Giuseppe, Prof.
(86) International application number: PCT/EP1998/003809
(87) International publication number: WO 1999/066906

(56) References cited:
- EP-A- 0 258 683
- GB-A- 2 185 187
- US-A- 5 736 154
- DATABASE WPI Week 9050 Derwent Publications Ltd., London, GB; AN 90-372979 XP002093942 & JP 02 270818 A (SEKISUI CHEM IND CO LTD) , 5 November 1990
- DATABASE WPI Week 9614 Derwent Publications Ltd., London, GB; AN 96-136203 XP002093912 & JP 08 027023 A (KATSU ET AL)

## Description

### Background of the Invention

The invention relates to a sticking plaster for controlled release of natural interferon for treating viral infections, in particular herpes infections. More particularly, the invention concerns an adhesive plaster for the controlled release of natural human α-interferon (nHuαIFN) derived from leucocytic or lymphoblastoid cells, for use in therapies designed to counter lip and genital herpes infections (caused by the viruses HSV-1 and HSV-2), Herpes Zoster (HZV), Zoster's neuritis (HZV) and flat verrucas (HPV).

For some time, nHuαIFN has been used in studies in the field of viral infections and has found a clearly-defined role in the last few years in therapies against acute and chronic viral pathologies, where it is often considered to be a "first choice drug".

The majority of the mechanism by which nHuαIFN acts is known depends on its direct interaction with the target cells and/or on the inducement of a biological response in the host organism.

The activity of the α-interferon as an immunomodulant plays an important role at the organic level in the defence mechanism against viral infections. nHuαIFN is administered parenterally (intravenously, intramuscularly, subcutaneously), orally and topically (ointments and eye-washes). The dosages usually recommended are in the order of millions of I.U. (International Units) for parenteral administrations, hundreds of thousands of I.U. for topical preparations and hundreds of I.U. for oral administrations. These administrations (excluding oral administrations) are prejudiced by a series of more or less serious drawbacks, which make interferon therapy unattractive to patients.

The most remarkable disturbances range from serious collateral effects, such as nausea, vomiting or obnubilation of the senses, which require the interruption of the therapy, to certain less significant ones, such as the troublesome repetition of the administrations and the greasiness of the ointments.

In addition, as a result of the rapid fall-off in the plasmatic concentrations of the active ingredient, it is sometimes necessary to resort to overdosage in order to achieve satisfactory pharmacological effects, running the risk of generating serious collateral effects and high therapy costs (the therapy is an assisted one practised with hospital or day hospital care).

There is therefore a need for a formulation designed to cope with the above-mentioned pathologies when diagnosed with certainty, which would enable the dose of nHuαIFN to be reduced, without altering its therapeutic effectiveness, and which could be used with a more pleasant method of administration and have stronger, more lasting pharmacological effects.

The authors of this invention have developed a sticking plaster for controlled release of nHuαIFN that overcomes the inconveniences of the known formulations.

### Summary of the Invention

In fact, the application of an occlusive medication and a system of controlled release enables very small doses of nHuαIFN to be administered in the very place where the viral lesion is situated.

The subject matter of the invention consists of a sticking plaster for the controlled release of natural human α-interferon for local transcutaneous absorption comprising: - a thin, inert, flexible support comprising an inner side and an outer side; - a layered adhesive on the inner side of said support so as to form a matrix; - a therapeutically effective quantity of said interferon dispersed in the matrix; in which said support is not repellent for said matrix, but is repellent for the interferon.

The support is preferably on a polyurethane base. The adhesive preferably comprises substances on an acrylic base.

The quantities of nHuαIFN present are preferably from 150 I.U. (International Units) to 150,000 I.U., more preferably from 150 I.U. to 1,500 I.U., 15,000 I.U. or 150,000 I.U., which in the doctors's opinion would enable the dosage to be varied. The sticking plaster normally has an applicatoin autonomy of 24 hours.

The sticking plaster should preferably comprise a release strip, as an element of protection of the inner side, which can be removed at the moment when it is applied, substantially of the same dimensions as the support and preferably manufactured with a central pre-cut.

It would be an advantage that the sticking plaster be available in various shapes, to facilitate its use in practice: a square shape and anatomic shapes for specific parts of the body.

### Preferred Embodiment

The invention will now be described for the purposes of illustration but not limitation, with reference to the method used to prepare a 3 x 3 cm sticking plaster. A sticking plaster for transcutaneous absorption, for controlled release of nHuαIFN derived from leucocytic or lymphoblastoid cells, is prepared as follows: on a plastic support with a square shape and rounded corners, measuring 3 x 3 cm, a matrix is spread that consists of: an acrylic base, acrylic acid, ethylenic glycol, a water alcohol solution and nHuαIFN of the type and dosage desired, which in its turn is covered with a removable protective layer (release strip) manufactured with a central pre-cut.

## Claims

1. A sticking plaster for the controlled release of natural human α-interferon for transcutaneous absorption, comprising:
- a thin, flexible support on the base of polyurethanes, comprising an inner side and an outer side;
- a layered adhesive on an acrylic base on the inner side of said support, so as to form a matrix;
- a therapeutically effective quantity of said interferon dispersed in said matrix;
in which said support is not repellent for said matrix, but is repellent for the interferon.

2. A sticking plaster according to claim 1 in which said interferon is contained in quantities ranging from 150 I.U. to 150,000 I.U.

3. A sticking plaster according to one of the preceding claims in which said interferon is contained in quantities of 150 I.U., 1,500 I.U., 15,000 I.U. or 150,000 I.U.

4. A sticking plaster according to one of the preceding claims further comprising a removable protective element for the inner side of said support, of substantially the same dimensions as the support.

5. The use of natural human α-interferon for preparing a sticking plaster for the controlled release of interferon for transcutaneous absorption, for use in anti-herpes therapies.

## Patentansprüche

1. Klebepflaster für die kontrollierte Freisetzung von natürlichem menschlichem α-Interferon zur transkutanen Absorption, mit:
- einem dünnen, flexiblen Träger auf Polyurethanbasis, der eine Innenseite und eine Außenseite hat;
- einem geschichteten Klebstoff auf Akrylbasis auf der Innenseite des Trägers zur Bildung einer Matrix;
- einer in der Matrix dispergierten therapeutisch wirksamen Menge des Interferons;
worin der Träger nicht für die Matrix, aber für das Interferon abstoßend ist.

2. Klebepflaster nach Anspruch 1, in welchem das Interferon in Mengen im Bereich von 150 IE bis 150.000 IE enthalten ist.

3. Klebepflaster nach einem der vorhergehenden Ansprüche, in welchem das Interferon in Mengen von 150 IE, 1.500 IE, 15.000 IE oder 150.000 IE enthalten ist.

4. Klebepflaster nach einem der vorhergehenden Ansprüche mit einem entfernbaren Schutzelement für die Innenseite des Trägers, das im Wesentlichen die gleichen Abmessungen hat wie der Träger.

5. Verwendung natürlichen menschlichen α-Interferons zur Herstellung eines Klebepflasters für die kontrollierte Interferonfreisetzung zur transkutanen Absorption für die Verwendung in Anti-Herpes-Therapien.

## Revendications

1. Sparadrap destiné à libérer de manière contrôlée de l'interféron alpha humain naturel en vue de son absorption transcutanée, comprenant:
- un support flexible mince à base de polyuréthanes comprenant un côté intérieur et un côté extérieur;
- un adhésif à base d'acrylique disposé en couche sur le côté intérieur dudit support, afin de former une matrice; et
- une quantité thérapeutiquement efficace dudit interféron dispersée dans ladite matrice;
dans lequel ledit support ne constitue pas un répulsif pour ladite matrice, mais constitue un répulsif pour l'interféron.

2. Sparadrap selon la revendication 1, dans lequel ledit interféron est contenu en des quantités qui se situent dans la plage de 150 U.I. à 150 000 U.I.

3. Sparadrap selon la revendication 1 ou 2, dans lequel ledit interféron est contenu en des quantités de 150 U.I., 1 500 U.I., 15 000 U.I. ou 150 000 U.I.

4. Sparadrap selon l'une quelconque des revendications précédentes comprenant également un élément protecteur amovible pour le côté intérieur dudit support, de dimensions sensiblement identiques à celles du support.

5. Utilisation d'interféron alpha humain naturel pour préparer un sparadrap destiné à libérer de manière contrôlée de l'interféron en vue de son absorption transcutanée, dans des thérapies anti-herpétiques.
